# FASCICULE DE BREVET EUROPEEN

(11) **EP 0 902 788 B1**
(45) Date de publication et mention de la délivrance du brevet: **19.03.2003**
(21) Numéro de dépôt: 97921911.0
(22) Date de dépôt: 30.04.1997
(51) Int. Cl.: C07H 19/06, C07H 19/10, C07H 19/16, C07H 19/20, C07H 21/00

(54) **DERIVES DE NUCLEOSIDES, ET LEUR UTILISATION POUR LA SYNTHESE D'OLIGONUCLEOTIDES**
NUKLEOSIDE-DERIVATE UND IHRE VERWENDUNG FÜR DIE SYNTHESE OLIGONUKLEOTIDEN
NUCLEOSIDE DERIVATIVES, AND THEIR USE IN OLIGONUCLEOTIDE SYNTHESIS

(30) Priorité: 03.05.1996 FR 9605553
(43) Date de publication de la demande: 24.03.1999
(73) Titulaire: CIS BIO INTERNATIONAL, 91400 Saclay (FR)
(72) Inventeur: TEOULE, Robert, F-38000 Grenoble (FR); ROGET, André, F-38120 Saint Egrève (FR); LIVACHE, Thierry, F-38560 Haute Jarrie (FR)
(74) Mandataire: Vialle-Presles, Marie José
(86) Numéro de dépôt international: FR9700770
(87) Numéro de publication internationale: WO97042207

(56) Documents cités:
- WO-A-94/22889
- JOURNAL OF ORGANIC CHEMISTRY, vol. 59, no. 10, 1994, EASTON US, pages 2715-2723, XP002025084 DE VOSS J J ET AL: "General Approach to the Synthesis of Specifically Deuterium-Labeled Nucleosides"
- JOURNAL OF MEDICINAL CHEMISTRY, vol. 15, no. 9, 1972, WASHINGTON US, pages 940-944, XP002025085 BAKER B R ET AL: "Irreversible enzyme inhibitors. 195. Inhibitors of thymidine kinase from Walker 256 carcinoma derived from thymidine 5'-acetate"
- BULLETIN OF THE CHEMICAL SOCIETY OF JAPAN, vol. 49, no. 11, 1976, TOKYO JP, pages 3357-3358, XP002025086 SHIMOKAWA S ET AL: "Studies on nucleosides and nucleotides. V. Selective aroylation of 5'-hydroxyl group of uridine and adenosine"
- TETRAHEDRON, vol. 50, no. 34, 1994, OXFORD GB, pages 10225-10234, XP002025087 MAG M ET AL: "Synthesis of dinucleotides containing a bridged non-chiral internucleotide 5'- or 3'-phosphoramidates linkage"

## Description

L'Invention est relative à des réactifs pour la synthèse oligonucléotidique, et en particulier à des dérivés de nucléosides et des réactifs de blocage utilisables pour la synthèse électrochimique d'oligonucléotides.

La demande PCT WO 94/22889 au nom de CIS BIO INTERNATIONAL (Inventeurs TEOULE et al.), décrit des dérivés de polymères conducteurs électroniques (PCE), utilisables comme support pour la synthèse de polymères biologiques, en particulier d'oligonucléotides. L'une des utilisations de dérivés de polymères conducteurs électroniques qui est décrite dans cette Demande est la synthèse adressée d'oligonucléotides *in situ* sur des microélectrodes recouvertes de polymère conducteur électronique, ce qui permet en particulier d'obtenir des matrices d'oligonucléotides. Les matrices d'oligonucléotides ou de peptides constituent en effet un outil très intéressant dans le domaine du séquençage, ainsi que dans le domaine du diagnostic (maladies génétiques, maladies infectieuses, cancer, typage cellulaire) ou pour le criblage de molécules actives.

Lors des procédés classiques de synthèse d'oligonucléotides sur support solide, on utilise des nucléotides dont le groupe phosphate, la fonction amine exocyclique des bases A, C, et G, et le groupe 5'-OH du sucre portent des groupements protecteurs. Pour effectuer chaque étape d'élongation de la chaîne oligonucléotidique, on déprotège le groupe 5'-OH du sucre du nucléoside à partir duquel on procède à l'élongation, de manière à ce que la fonction 5'-OH libérée puisse réagir, en présence d'un agent de couplage, avec le groupe phosphate du nucléotide suivant pour former une liaison phosphotriester (éventuellement par l'intermédiaire d'un phosphite qui est ensuite converti en phosphotriester par oxydation). L'on répète ensuite le cycle : déprotection du groupe 5'-OH/couplage du nucléotide suivant par l'intermédiaire d'une liaison phosphotriester, jusqu'à obtention de l'oligonucléotide de la taille souhaitée. A la fin de chaque cycle, on procède au blocage, par estérification, des fonctions 5'-OH n'ayant pas réagi ; cette étape est dénommée « coiffage » (capping).

La protection des bases A, C, et G a pour but d'éviter des réactions parasites impliquant les fonctions amine exocycliques de ces bases. Les groupements protecteurs de ces bases, ainsi que ceux du groupe phosphate, sont coupés à la fin de la synthèse de l'oligonucléotide.

L'utilisation d'un polymère conducteur électronique comme support de synthèse permet de procéder à la synthèse d'oligonucléotides en mettant en oeuvre une ou plusieurs étapes de coupure électrochimique des différents groupes protecteurs. Cette déprotection électrochimique est assurée en créant une différence de potentiel entre l'électrode de travail et une contre-électrode.

L'utilisation de microélectrodes disposées en matrice comme support solide pour la synthèse *in situ* d'oligonucléotides permet de procéder, pour l'addition d'un nucléotide déterminé, à la déprotection électrochimique sélective de la fonction 5'-OH des chaînes d'oligonucléotides en croissance sur certaines des électrodes de la matrice ; ceci permet donc de n'ajouter le nucléotide concerné qu'aux chaînes oligonucléotidiques souhaitées, et d'obtenir, en fin de synthèse des matrices d'oligonucléotides portant, en des points différents, des oligonucléotides de séquence différentes.

La demande PCT WO 94/22889 citée ci-dessus, décrit ainsi l'utilisation d'un groupement thiopixyle ou d'un groupement *p*-nitrobenzoyle en tant que groupement protecteur électrolabile de la fonction 5'-OH du sucre.

Pour être en mesure de synthétiser des oligonucléotides de séquences variées, en mettant en oeuvre une ou plusieurs étapes de déprotection électrochimique, il convient d'utiliser, pour toutes les fonctions à protéger, des groupements protecteurs compatibles entre eux.

C'est ainsi que le groupement protecteur électrolabile de la fonction 5'-OH du sucre doit pouvoir être coupé à un potentiel qui n'affecte ni la stabilité des nucléosides, ni celle des groupements protecteurs de la fonction aminé exocyclique des bases A, C, et G. En ce qui concerne ces derniers, ils ne doivent pas modifier les propriétés électroniques de la base, ce qui risquerait d'influer sur sa réactivité ou la stabilité de la liaison N-glycosidique. Il faut donc sélectionner des groupements protecteurs qui ne déstabilisent pas les nucléosides vis-à-vis des réactifs de la synthèse oligonucléotidique, ou vis-à-vis des espèces réactives générées lors de l'application du potentiel de déprotection, et qui en outre, permettent une solubilité suffisante du nucléoside.

De même, le groupement protecteur du phosphore en 3' doit être stable dans les conditions utilisées pour la déprotection électrochimique du sucre et des bases.

Enfin, l'ester formé lors du coiffage pour bloquer les fonctions 5'-OH n'ayant pas réagi doit également être stable dans ces conditions.

Les Inventeurs ont maintenant mis au point des dérivés de nucléosides portant des groupements protecteurs répondant à l'ensemble des conditions mentionnées ci-dessus, et en particulier permettant de procéder aisément à une déprotection électrochimique lors de la synthèse d'oligonucléotides. La présente Invention a pour objet l'utilisation, dans un procédé de synthèse d'oligonucléotides comprenant au moins une étape électrochimique, d'au moins un dérivé de nucléoside répondant à la formule (I) ci après : 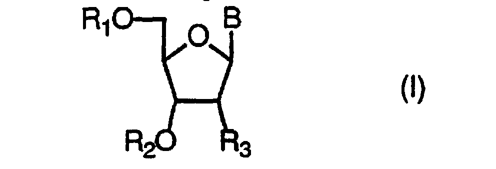 dans laquelle
- R1 représente :
   * un groupement électrolabile répondant à la formule (IIa) suivante : 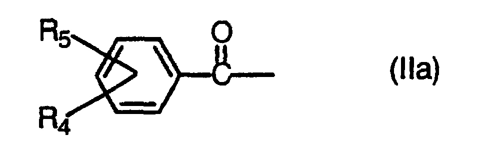
   dans laquelle R4 représente un groupement NO₂ et R5 représente un atome d'hydrogène, un groupe alkyle en C₁ à C₁₀ linéaire ou ramifié, un groupe 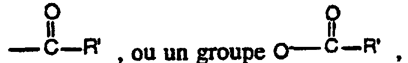 où R' représente un groupe alkyle en C₁ à C₁₀ linéaire ou ramifié, ou un groupe ―O―R' où R' représente un groupe alkyle en C₁ à C₁₀ linéaire ou ramifié, ou bien
   un groupement électrolabile répondant à la formule (IIb) suivante : 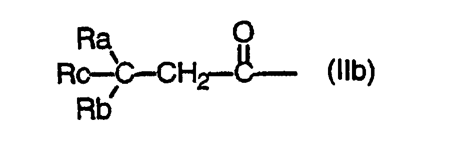 dans laquelle l'un des groupes Ra, Rb, ou Rc représente un groupe phényle, ou un groupe phényle substitué, et les deux autres représentent des atomes d'hydrogène,
- R2 représente un atome d'hydrogène, ou un groupe phosphoroamidite, phosphonate, ou phosphotriester ;
- R3 représente un atome d'hydrogène, un radical hydroxyle, un radical hydroxyle substitué, ou un halogène.
- B représente un radical dérivé d'une base purique ou pyrimidique répondant à l'une des formules suivantes : 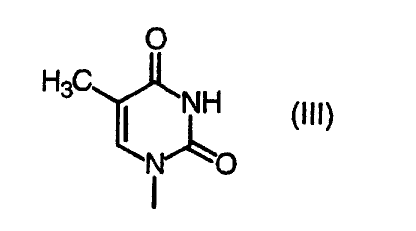 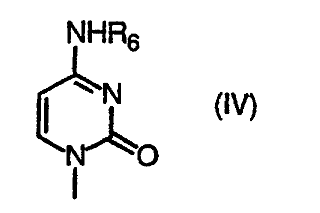 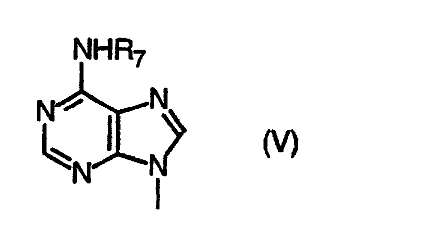 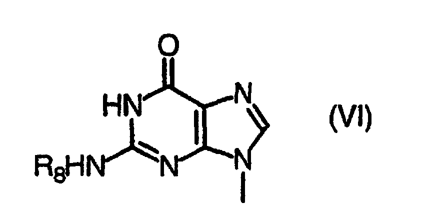 où R6 représente un groupement protecteur de la fonction amine, autre qu'un groupe isobutyryle ou phénylpropionyle, répondant à l'une des formules (VII) (VIII) et (IX) ci-dessous: 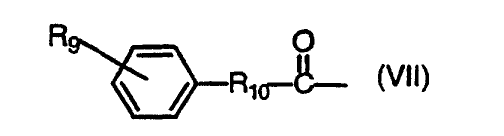 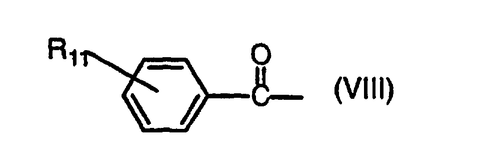 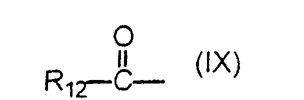 dans lesquelles R9 et R11 qui peuvent être identiques ou différents. représentent un atome d'hydrogène, ou un groupe alkyle ou alcoxy en C₁-C₁₀, R12 représente un radical alkyle linéaire ou ramifié en C₁-C₁₀, et R10 est choisi parmi les radicaux de formule

   -CH₂- ;

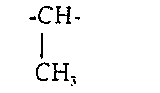

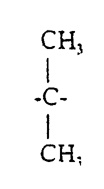

   -O-(CH₂)-

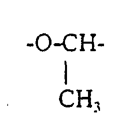

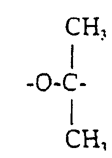

   * R7 représente un groupement protecteur de la fonction amine. autre qu'un groupe phénylpropionyle, répondant à l'une des formules (VII) (VIII) et (IX) définies ci-dessus ;
   * R8 représente un groupement protecteur de la fonction amine, autre qu'un groupe isobutyryle, phénylpropionyle, ou phénoxyacétyle, répondant à l'une des formules (VII) (VIII) et (IX) définies ci-dessus ;
   à l'exception des dérivés dans lesquels R1 est un groupement électrolabile de formule (IIa) dans laquelle R4 représente un groupement NO₂ et R5 un atome d'hydrogène, et B représente la thymine.

Des dérivés préférés sont ceux dans lesquels R1 répond à la formule (IIa) dans laquelle R4 représente un groupement NO₂ et R5 représente un atome d'hydrogène, et parmi ceux-ci, des dérivés dans lesquels :
- B répond à la formule IV dans laquelle R6 est un groupement anisoyle, ou bien
- B répond à la formule V dans laquelle R7 est un groupement benzoyle ou un groupement phénoxyacétyle, ou bien
- B répond à la formule VI dans laquelle R8 est un groupement *t*-butylphénoxyacétyle.

Les dérivés de nucléosides mentionnés ci-dessus peuvent subir sans altération toutes les opérations de la synthèse oligonucléotidique, et en particulier l'application du potentiel électrique nécessaire à la coupure des groupements protecteurs électrolabiles.

Ils sont donc particulièrement appropriés pour l'utilisation pour la synthèse d'oligonucléotides sur des supports recouverts de polymères conducteurs électroniques, en particulier des microélectrodes, ou des matrices de microélectrodes.

On peut aussi, de la même manière, utiliser ces dérivés de nucléosides pour la synthèse d'analogues structuraux des acides nucléiques (antisens, PNA...).

L'invention englobe également de nouveaux dérivés de nucléosides, qui peuvent être utilisés pour la synthèse d'oligonucléotides conformément à l'invention. Il s'agit des dérivés de formule générale (I) telle que définie ci-dessus, à l'exception du dérivé dans lequel :
- R1 est un groupement électrolabile de formule (IIa) dans laquelle R4 représente un groupement NO₂ et R5 un atome d'hydrogène, et B répond à la formule (III), et du dérivé dans lequel :
   - R1 est un groupement électrolabile de formule (IIa) dans laquelle R4 représente un groupement NO₂ et R5 un atome d'hydrogène, et B répond à la formule (IV) dans laquelle R6 est un groupe benzoyle.

La présente Invention a également pour objet un procédé de synthèse oligonucléotidique par addition successive de nucléotides à partir d'un premier nucléoside fixé sur un support solide, chaque addition d'un nucléotide comprenant la déprotection de la fonction 5'-OH du nucléotide ou du nucléoside précédent, et le couplage du nucléotide à ajouter, sur ladite fonction 5'-OH déprotégée, lequel procédé est caractérisé en ce qu'au moins une des étapes d'addition d'un nucléotide est effectuée en utilisant conformément à l'invention un dérivé de nucléoside tel que défini ci-dessus, et met en oeuvre une déprotection électrochimique de la fonction 5'-OH dudit dérivé de nucléoside.

Selon un mode de mise en oeuvre préféré du procédé conforme à l'Invention, le support solide est constitué par au moins une électrode recouverte d'un polymère conducteur électronique ; avantageusement, il s'agit d'un copolymère d'un dérivé de nucléoside et de polymère conducteur électronique tel que ceux définis dans la Demande PCT WO 94/22889.

Avantageusement, le support solide est constitué par plusieurs électrodes juxtaposées, et, si on le souhaite, la déprotection électrochimique de la fonction 5'-OH précédant le couplage d'un nucléotide déterminé, n'est effectuée que sur certaines de ces électrodes.

Selon un autre mode de mise en oeuvre préféré du procédé conforme à l'Invention, la déprotection électrochimique de la fonction 5'-OH est effectuée en appliquant à l'électrode portant le support solide de synthèse une différence de potentiel, comprise entre -1,6 V et -0,7 V par rapport à une électrode de référence Ag/AgNO₃.

Selon un autre mode de mise en oeuvre du procédé conforme à l'Invention, on effectue, après chaque étape de couplage d'un nucléotide, un traitement de coiffage par l'anhydride isobutyrique, afin de bloquer les fonctions 5'-OH n'ayant pas réagi à l'issue de l'étape de couplage. L'utilisation de l'anhydride isobutyrique permet la formation d'un ester stable dans les conditions de déprotection de la fonction 5'-OH.

La présente Invention sera mieux comprise à l'aide du complément de description qui va suivre, qui se réfère à des exemples de mise en oeuvre du procédé conforme à l'Invention.

Il doit être bien entendu toutefois que ces exemples sont donnés uniquement à titre d'illustration de l'objet de l'Invention dont ils ne constituent en aucune manière une limitation.

### EXEMPLE 1: PREPARATION DU DERIVE DE LA DESOXYCYTIDINE

### 1. CHOIX DES PROTECTIONS FONCTIONNELLES

Des essais de solubilité préalables ont permis d'éliminer le groupement isobutyryle, pour la protection de la fonction amine de la désoxycytidine, du fait de la mauvaise solubilité de la 5'*p*-nitrobenzoyl-4-N-isobutyryl désoxycytidine dans les solvants organiques. Le groupement phénylpropionyle est intéressant car la 5'*p*-nitrobenzoyl-4-N-phénylpropionyl désoxycytidine est très soluble dans les solvants organiques. Le groupement anisoyle confère également au dérivé 5'-nitrobenzoylé de la désoxycytidine une meilleure solubilité que le groupement benzoyle.

Les essais de synthèse avec déprotection électrochimique n'ayant pas donné de bons résultats en ce qui concerne la N-phénylpropionyl désoxycytidine, la stabilité du nucléoside protégé par la N-anisoyl désoxycytidine a été vérifiée. Les nucléosides ont été dissous dans le perchlorate de tétrabutylammonium 0,1 M dans le méthanol. Ils sont été soumis à un potentiel de -1,35 V pendant 30 minutes. L'analyse du mélange réactionnel a montré que la coupure du groupement phénylpropionyle est complète dans ces conditions. Le groupement anisoyle n'est quant à lui, pas coupé. Il a donc été décidé de remplacer la N-phénylpropionyl désoxycytidine par la N-anisoyl désoxycytidine et de préparer le phosphoramidite correspondant pour la synthèse *in-situ.*

### 2. SYNTHESE DU NUCLEOSIDE

La synthèse de la 5'-*p*-nitrobenzoyl-4-*N*-anisoyl désoxycytidine a été réalisée par réaction du chlorure de *p*-nitrobenzoyle (1,1eq) sur la 4-N-anisoyl-désoxycytidine (leq) dans la pyridine. Après extraction par un mélange dichlorométhane/bicarbonate de sodium, le produit a été obtenu par chromatographie sur colonne de silice. L'élution est faite par 4% de méthanol dans le dichlorométhane. Ce nucléoside est assez soluble dans les solvants organiques.

### 3. PREPARATION DE L'AMIDITE DE LA DESOXYCYTIDINE

Le nucléoside (leq) est séché dans un mélange dichlorométhane/acétonitrile et mis en réaction avec 1,2 eq de bisdiisopropylaminocyanoéthoxyphosphine et 0,5 eq de tétrazolure de diisopropylammonium dans le dichlorométhane anhydre. Après 2 heures de réaction le mélange réactionnel est dilué et lavé successivement par du bicarbonate de sodium 0,5 M et du chlorure de sodium saturé. La phase organique est évaporée à sec. Après redissolution dans le chloroforme, le produit est précipité dans l'hexane, séché sous vide et stocké sous argon. Cet amidite est soluble dans l'acétonitrile, solvant dans lequel sont classiquement réalisées les synthèses d'oligonucléotides.

### EXEMPLE 2 : PREPARATION DU DERIVE DE LA DESOXYADENOSINE

### 1) CHOIX DES PROTECTIONS FONCTIONNELLES

Les deux dérivés de la désoxyadénosine habituellement utilisés en synthèse d'oligonucléotides, à savoir le dérivé benzoylé (habituellement, déprotection 16 heures à 55°C), et le dérivé phénoxyacétylé (habituellement, déprotection 16 heures à température ambiante ou 4 heures à 55°C) ont été préparés.

### 2) SYNTHESE DE LA 5'-O-p-NITROBENZOYL-6-N-BENZOYL DESOXYADENOSINE

La synthèse de ce produit se fait par réaction de 1 eq de chlorure de *p*-nitrobenzoyle sur la 6-N-benzoyldésoxyadénosine 4 heures dans la pyridine. Après hydrolyse de l'excès de réactif, évaporation partielle de la pyridine, extraction et évaporation de la phase chloroformique, le produit est obtenu par chromatographie sur colonne (élution 4% de méthanol).

### 3) SYNTHESE DE LA 5'-O-p-NITROBENZOYL-6-N-PHENOXYACETYL DESOXYADENOSINE

La synthèse de ce produit se fait par réaction de 1,1 eq de chlorure de *p*-nitrobenzoyle sur la 6-N-phénoxyacétyl désoxyadénosine 4 heures dans la pyridine. Après hydrolyse de l'excès de réactif, évaporation presque totale de la pyridine, extraction acétate d'éthyle/bicarbonate de sodium, la phase acétate d'éthyle est évaporée à sec. Le produit est obtenu par chromatographie sur colonne avec un gradient de méthanol dans le chloroforme (élution 4% de méthanol).

### 4) RESULTAT

Les deux dérivés présentent une solubilité convenable. Le choix se portera sur le nucléoside phénoxyacétylé en raison de ses conditions de déprotection électrochimique plus favorables.

### 5) PREPARATION DE L'AMIDITE DE LA DESOXYADENOSINE

La 5'-O-*p*-nitrobenzoyl-6-N-phénoxyacétyl désoxyadénosine (1 eq) est séchée dans un mélange dichlorométhane/acétonitrile et mise en réaction avec 1,2 eq de bisdiisopropylaminocyanoéthoxyphosphine et 0,5 eq de tétrazolure de diisopropylammonium dans le dichlorométhane anydre. Après 2 heures de réaction, le mélange réactionnel est dilué et lavé successivement par du bicarbonate de sodium 0,5 M et du chlorure de sodium saturé. La phase organique est évaporée à sec. Après précipitation dans l'hexane, le produit est séché sous vide et stocké sous argon. Cet amidite est soluble dans l'acétonitrile, solvant dans lequel sont classiquement réalisées les synthèses d'oligonucléotides.

### EXEMPLE 3 : PREPARATION DU DERIVE DE LA DESOXYGUANOSINE

### 1) CHOIX DU GROUPEMENT PROTECTEUR

Les essais de protection de la désoxyguanosine sur sa fonction amine en position 2, ont été effectués avec les groupements suivants : isobutyryle, phénoxyacétyle et *t*-butylphénoxyacétyle.

### 2) SYNTHESE DE LA 5'-O-p-NITROBENZOYL-2-N-ISOBUTYRYL DESOXYGUANOSINE

La synthèse de ce produit se fait par réaction de 1,1 eq de chlorure de *p*-nitrobenzoyle sur la 2-N-isobutyryl désoxyguanosine, pendant 16 heures dans la pyridine. Après extraction par un mélange dichlorométhane/bicarbonate de sodium et évaporation à sec, le produit est repris par du chloroforme. Ce produit n'a pas été retenu car trop insoluble.

### 3) SYNTHESE DE LA 5'-O-p-NITROBENZOYL-2-N-PHENOXYACETYL DESOXYGUANOSINE

La synthèse de ce produit se fait par réaction de 1,1 eq de chlorure de *p*-nitrobenzoyle sur la 2-N-phénoxyacétyl désoxyguanosine, pendant 16 heures dans la pyridine. Après extraction par un mélange dichlorométhane/bicarbonate de sodium et évaporation à sec, le produit est repris par du chloroforme et purifié sur colonne de silice (élution 4% de méthanol). Le produit est peu soluble dans les solvants organiques.

### 4) SYNTHESE DE L'AMIDITE DE LA 5'-O-p-NITROBENZOYL-2-N-PHENOXYACETYL DESOXYGUANOSINE

Le nucléoside est séché par coévaporation dans un mélange dicholorométhane/acétonitrile et mis en réaction avec 1,2 eq de bisdiisopropylamino cyanoéthoxyphosphine et 0,5 eq de tétrazolure de diisopropylammonium dans le dichlorométhane anhydre (produit non soluble). Après 2 heures de réaction, le mélange réaction est dilué et lavé successivement par du bicarbonate de sodium 0,5 M et du chlorure de sodium saturé. La phase organique est évaporée à sec. Après précipitation dans l'hexane le produit est séché sous vide et stocké sous argon. L'amidite est soluble dans le dichlorométhane. Il est difficilement soluble dans l'acétonitrile et l'on observe un dépôt dans le flacon au bout de moins d'une heure. Un dérivé plus soluble a été préparé en substituant le groupement phénoxyacétyle.

### 5) SYNTHESE DE LA 2-N-t-BUTYLPHENOXYACETYL DESOXYGUANOSINE

La synthèse de ce produit a nécessité la préparation de chlorure de *t*-butylphénoxyacétyle à partir de *t*-butylphénolate de sodium et du chloracétate d'éthyle. Le *t*-butylphénoxyacétate d'éthyle obtenu est coupé par la potasse afin de libérer l'acide *t*-butylphénoxyacétique. Le chlorure d'acide est obtenu par chauffage de cet acide dans le chlorure de thionyle. Après élimination de l'excès de chlorure de thionyle le chlorure de *t*-butylphénoxyacétyle est mis en réaction avec la désoxyguanosine suivant le mode opératoire décrit par SCHULHOF et al. pour la 2-N-phénoxyacétyl-désoxyguanosine [Nucleic Acids Res., 15(2), p. 397-415, (1987)].

### 6) SYNTHESE DE LA 5'-O-p-NITROBENZOYL-2-N-t-BUTYL-PHENOXYACETYL DESOXYGUANOSINE

La synthèse de ce produit se fait par réaction de 1,1 eq de chlorure de *p*-nitrobenzoyle sur la 2-N-*t*-butylphénoxyacétyl désoxyguanosine, pendant 16 heures, dans la pyridine. Après extraction par un mélange dichlorométhane/méthanol et évaporation à sec, le produit est repris par chloroforme/hexane (8/2) et purifié sur colonne de silice (élution 3% de méthanol). Le produit obtenu est très soluble dans les solvants organiques.

### 7) RESULTAT

Les dérivés usuels de la désoxyguanosine n'étant pas assez solubles il a été nécessaire de recourir au groupement *t*-butylphénoxyacétique. Ce groupement a permis de préparer un dérivé de la désoxyguanosine très soluble et portant lui aussi un groupement protecteur qui est coupé dans des conditions douces.

### 8) PREPARATION DE L'AMIDITE DE LA DESOXYGUANOSINE

La 5'-O-*p*-nitrobenzoyl-2-N-*t*-butylphénoxy-acétyl désoxyguanosine (1 eq) est séchée dans un mélange dichlorométhane/acétonitrile et mise en réaction avec 1,2 eq de bisdiisopropylaminocyanoéthoxyphosphine et 0,5 eq de tétrazolure de diisopropylammonium dans le dichlorométhane anhydre. Après 2 heures de réaction, le mélange réactionnel est dilué et lavé successivement par du bicarbonate de sodium 0,5 M et du chlorure de sodium saturé. La phase organique est évaporée à sec. Après redissolution dans le chloroforme et précipitation dans l'hexane, le produit est séché sous vide et stocké sous argon. Cet amidite est soluble dans l'acétonitrile, solvant dans lequel sont classiquement réalisées les synthèses d'oligonucléotides.

### EXEMPLE 4 : PREPARATION DU SUPPORT GREFFE CLIVABLE

### 1) PRINCIPE

Le support greffé a été préparé par copolymérisation de pyrrole et de nucléoside pyrrole. Afin de pouvoir analyser l'oligonucléotide formé, le nucléoside et le pyrrole sont reliés par l'intermédiaire d'une fonction ester qui sera coupée par l'ammoniaque lors de la déprotection de l'oligonucléotide. Le nucléoside est protégé en 5' par un groupe *p*-nitrobenzoyle, qui est stable dans les conditions utilisées pour la polymérisation, afin de permettre le blocage du support avant synthèse.

### 2) PREPARATION DU NUCLEOSIDE SUCCINYLE

La *p*-nitrobenzoylthymidine (10 mmoles) est séchée par coévaporation dans la pyridine. On reprend par 10 ml de pyridine et on ajoute de l'anhydre succinique (12 mmoles) et la DMAP (12 mmoles). La réaction est effectuée pendant 16 heures à température ambiante. Après extraction dichlorométhane/bicarbonate de sodium, le mélange réactionnel est évaporé à sec, repris par du dichlorométhane, puis purifié sur colonne de silice.

### 3) PREPARATION DU NUCLEOSIDE PYRROLE

La 5'-*p*-nitrobenzoylthymidine succinylée (2 mmoles) est séchée par coévaporation dans la pyridine anhydre et reprise par 20 ml d'acétonitrile. On ajoute du dicyclohexylcarbodiimide (2,2 mmoles) et de l'aminoéthyl-pyrrole (2 mmoles). La réaction est effectuée pendant 16 heures. Le précipité de DCU (Dicyclohexylurée) est éliminé par filtration. Le mélange réactionnel est évaporé et repris par 100 ml de dichlorométhane. Après extraction par le dichlorométhane/bicarbonate de sodium, et évaporation de la phase organique, le produit est obtenu par chromatographie sur colonne de silice avec un gradient de méthanol dans le chloroforme.

### 4) COPOLYMERISATION AVEC LE PYRROLE

Le nucléoside couplé au pyrrole 10⁻³ M, et le pyrrole 10⁻² M, sont copolymérisés en solution dans le perchlorate de tétrabutylammonium 0,1 M dans l'acétonitrile. La synthèse se fait par balayage entre -0,35 V et +0,85 V par rapport à Ag/AgNO₃. La cellule électrochimique est composée d'un tube de polypropylène dans lequel plongent une électrode et une contre-électrode constituées toutes deux d'un ruban de platine de 0,3 x 1 cm et une électrode de référence (Ag/AgNO₃). Lors du balayage le copolymère de pyrrole se dépose sous forme d'un film noir adhérant sur l'électrode.

Le support ainsi obtenu peut être utilisé pour la synthèse d'oligonucléotides avec déprotection électrochimique lorsque l'on veut récupérer l'oligonucléotide à des fins d'analyse après la fin de la synthèse.

### EXEMPLE 5: SYNTHESE IN-SITU SUR SUPPORT CLIVABLE DE L'OLIGONUCLEOTIDE AAAAT

Le premier nucléoside en 3' a été introduit lors de la copolymérisation qui a servi à préparer le support : c'est une thymidine protégée par un *p*-nitrobenzoyle sur son hydroxyle en 5'. Ce premier nucléoside est déprotégé pendant 15 minutes à -1.35 V dans Bu₄N⁺ClO₄⁻ 0.1 M dans le méthanol. L'électrode est déconnectée et placée dans une colonne de synthèse. Il est alors procédé aux étapes suivantes du cycle (condensation. blocage, oxydation sur le synthétiseur). La condensation se fait avec l'amidite A protégé par un *p*-nitrobenzoyle sur sa fonction hydroxyle en 5' et un groupement phénoxyacétyle sur la fonction amine de la base nucléique. Le phosphite est oxydé par une solution d'iode. Pour le blocage des fonctions 5'-OH n'ayant pas réagi, on utilise l'anhydride isobutyrique à 10% dans le THF avec un temps de contact de 30 secondes. En effet l'anhydrique acétique ne convient pas pour le blocage car l'ester formé est coupé lors de la déprotection électrochimique.

L'électrode est sortie de la colonne et placée dans la cellule d'électrochimie. Le groupement *p*-nitrobenzoyle est coupé par application d'un potentiel de -1,35 V, pendant 15 minutes. L'électrode est déconnectée puis replacée dans la colonne de synthèse. On procède aux étapes chimiques : condensation avec l'amidite A protégé comme indiqué ci-dessus, blocage et oxydation. Ce cycle alternant une étape de déprotection électrochimique et trois étapes chimiques, est poursuivi en introduisant encore trois fois l'amidite A jusqu'à l'obtention de AAAAT portant des fonctions amines protégées.

A la fin de la synthèse, l'oligonucléotide obtenu est coupé du support et les groupes protecteurs des fonctions amines sont clivés par chauffage dans l'ammoniaque durant 16 heures.

### EXEMPLE 6: SYNTHESE IN-SITU SUR SUPPORT CLIVABLE DE L'OLIGONUCLEOTIDE CCCCT

La synthèse est réalisée suivant le mode opératoire décrit pour AAAAT en replaçant à chaque cycle l'amidite de la 5'*p*-nitrobenzoyl-N-phénoxyacétyldésoxyadénosine par celui de la 5'*p*-nitrobenzoyl-4-N-anisoyl-désoxycytidine.

A la fin de la synthèse l'oligonucléotide obtenu est coupé du support et les groupes protecteurs des fonctions amines sont clivés par chauffage dans l'ammoniaque durant 16 heures.

### EXEMPLE 7: SYNTHESE IN-SITU SUR SUPPORT CLIVABLE DE L'OLIGONUCLEOTIDE GGGGT

La synthèse est réalisée suivant le mode opératoire décrit pour AAAAT en replaçant à chaque cycle l'amidite de la 5'*p*-nitrobenzoyl-N-phénoxyacétyl désoxyadénosine par celui de la 5'*p*-nitrobenzoyl-2-N-*t*-butyl-phénoxyacétyldésoxyguanosine.

A la fin de la synthèse l'oligonucléotide obtenu est coupé du support et les groupes protecteurs des fonctions amines sont clivés par chauffage dans l'ammoniaque durant 16 heures.

### EXEMPLE 8 : PREPARATION D'UN SUPPORT GREFFE NON CLIVABLE

### 1) PRINCIPE

Pour réaliser la synthèse de l'oligonucléotide sur le support, il faut introduire sur la matrice une fonction susceptible de se coupler avec un phosphoramidite. Cette fonction, qui servira de point d'ancrage pour la synthèse *in-situ* de l'oligonucléotide, sera avantageusement introduite lors de la copolymérisation. Il est intéressant d'introduire un dérivé du pyrrole portant un bras espaceur, ayant à son extrémité une fonction réactive vis-à-vis du phosphoramidite, cette fonction étant protégée par un groupement électrolabile.

Afin de pouvoir conserver sur la matrice l'oligonucléotide formé, le premier nucléoside et le pyrrole sont reliés par l'intermédiaire d'une fonction phosphodiester qui sera donc stable lors de la déprotection de l'oligonucléotide par l'ammoniaque. Ce réactif est protégé afin de permettre le blocage du support avant synthèse par le groupe *p*-nitrobenzoyle qui est stable dans les conditions utilisées pour la polymérisation.

### 2) NITROBENZOYLATION DU PYRROLE TRIETHYLENEGLYCOL (PYTEG)

Le PYTEG (10 mmoles, 2 g) est séché par coévaporation dans la pyridine anhydre. On reprend par 100 ml de pyridine et on ajoute le chlorure de nitrobenzoyle (11 ml, 2 g). Après 4 à 16 heures de réaction le solvant est éliminé et le mélange réactionnel est repris par 100 ml de chloroforme. La solution est lavée par 3 x 100 ml de NaHCO₃ saturé. Les phases aqueuses sont contre-extraites par 50 ml de chloroforme. Les phases organiques sont regroupées et évaporées à sec. Le résidu est repris par 5 ml de chloroforme et séparé sur une colonne de silice conditionnée dans le chloroforme. Le produit est élué avec 5% de méthanol dans le chloroforme. On obtient 2,97 g de produit (85% de rendement).

### 3. COPOLYMERISATION DU PYTEG AVEC LE PYRROLE SUR UNE ELECTRODE.

Le PYTEG couplé au nitrobenzoyle 10⁻³ M, et le pyrrole 10⁻² M ont été copolymérisés en solution dans le perchlorate de tétrabutylammonium 0,1 M dans l'acétonitrile. La synthèse se fait par balayage entre -0,35 V et +0,85 V par rapport à Ag/AgNO₃. La cellule électrochimique est composée d'un tube de polyéthylène dans lequel plongent une électrode et une contre-électrode constituées toutes deux d'un ruban de platine de 0,5 x 1 cm, et une électrode de référence (Ag/AgNO₃). Lors du balayage, le copolymère se dépose sous forme d'un film adhérent sur l'électrode. Ce support sera utilisé pour la synthèse d'oligonucléotides avec déprotection électrochimique lorsque l'on ne veut pas couper l'oligonucléotide après la fin de la synthèse.

## Revendications

1. Utilisation, dans un procédé de synthèse d'oligonucléotides comprenant au moins une étape de déprotection électrochimique et antérieurement à ladite étape de déprotection, d'au moins un dérivé de nucléoside répondant à la formule (I): 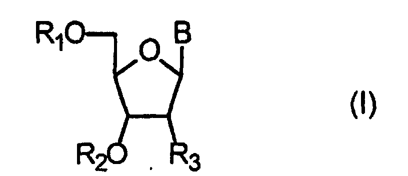 dans laquelle
- R1 représente :
* un groupement électrolabile répondant à la formule (IIa) suivante : 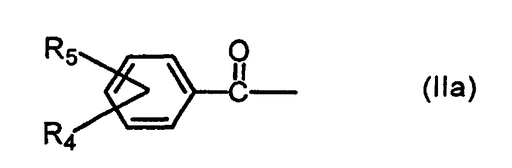 dans laquelle R4 représente un groupement NO₂ et R5 représente un atome d'hydrogène, un groupe alkyle en C₁ à C₁₀ linéaire ou ramifié, un groupe 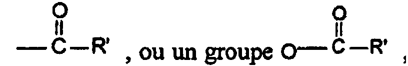 , où R' représente un groupe alkyle en C₁ à C₁₀ linéaire ou ramifié, ou un groupe ―O―R' où R' représente un groupe alkyle en C₁ à C₁₀ linéaire ou ramifié, ou bien
un groupement électrolabile répondant à la formule (IIb) suivante : 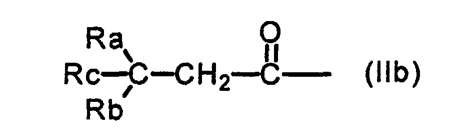 dans laquelle l'un des groupes Ra, Rb, ou Rc représente un groupe phényle, ou un groupe phényle substitué, et les deux autres représentent des atomes d'hydrogène
- R2 représente un atome d'hydrogène ou un groupe phosphoroamidite, phosphonate, ou phosphotriester ;
- R3 représente un atome d'hydrogène, un radical hydroxyle, un radical hydroxyle substitué, ou un halogène.
- B représente un radical dérivé d'une base purique ou pyrimidique répondant à l'une des formules suivantes : 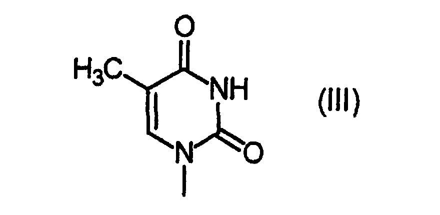 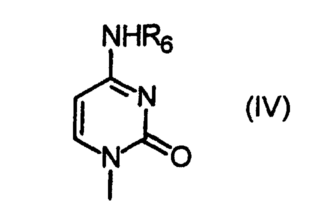 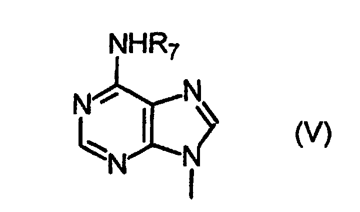 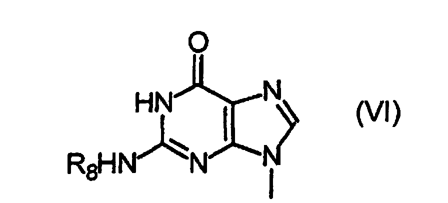 où R6 représente un groupement protecteur de la fonction amine, autre qu'un groupe isobutyryle ou phénylpropionyle, répondant à l'une des formules (VII) (VIII) et (IX) ci-dessous: 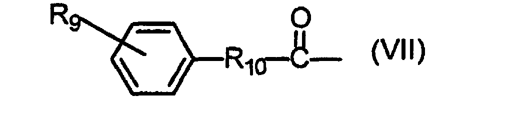 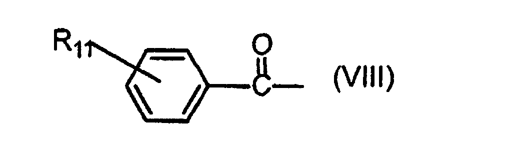 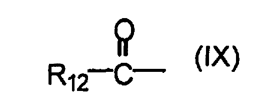 dans lesquelles R9 et R11 qui peuvent être identiques ou différents, représentent un atome d'hydrogène, ou un groupe alkyle ou alcoxy en C₁-C₁₀, R12 représente un radical alkyle linéaire ou ramifié en C₁-C₁₀, et R10 est choisi parmi les radicaux de formule :
-CH₂-; 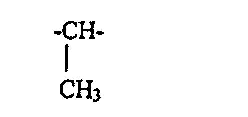 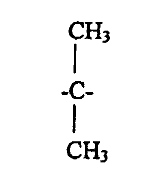
-O-(CH₂)- 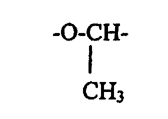 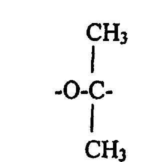
* R7 représente un groupement protecteur de la fonction amine, autre qu'un groupe phénylpropionyle, répondant à l'une des formules (VII) (VIII) et (IX) définies ci-dessus ;
* R8 représente un groupement protecteur de la fonction amine, autre qu'un groupe isobutyryle, phénylpropionyle, ou phénoxyacétyle, répondant à l'une des formules (VII) (VIII) et (IX) définies ci-dessus ;
à l'exception des dérivés dans lesquels R1 est un groupement électrolabile de formule (IIa) dans laquelle R4 représente un groupement NO₂ et R5 un atome d'hydrogène, et B répond à la formule (III).

2. Utilisation selon la revendication 1 **caractérisée en ce que** l'on met en oeuvre un dérivé dans lequel R1 répond à la formule (IIa), dans laquelle R4 représente un groupement NO₂ et R5 représente un atome d'hydrogène.

3. Utilisation selon la revendication 2 **caractérisée en ce que** l'on met en oeuvre un dérivé dans lequel B répond à la formule (IV) dans laquelle R6 est un groupement anisoyle.

4. Utilisation selon la revendication 2 **caractérisée en ce que** l'on met en oeuvre un dérivé dans lequel B répond à la formule (V) dans laquelle R7 est un groupement benzoyle ou un groupement phénoxyacétyle.

5. Utilisation selon la revendication 2 **caractérisée en ce que** l'on met en oeuvre un dérivé dans lequel B répond à la formule (VI) dans laquelle R8 est un groupement *t*-butylphénoxyacétyle.

6. Dérivé de nucléosides tel que défini dans une quelconque des revendications 1 à 5, à l'exception des dérivés dans lesquels R1 est un groupement électrolabile de formule (IIa) dans laquelle R4 représente un groupement NO₂ et R5 un atome d'hydrogène, et B répond soit à la formule (III), soit à la formule (IV) dans laquelle R6 est un groupe benzoyle.

7. Procédé de synthèse oligonucléotidique par addition successive de nucléotides à partir d'un premier nucléoside fixé sur un support solide, chaque addition d'un nucléotide comprenant la coupure du groupe protecteur de la fonction 5'-OH du nucléotide ou du nucléoside précédent, et le couplage du nucléotide à ajouter. sur ladite fonction 5'-OH déprotégée, lequel procédé est **caractérisé en ce qu'**au moins une des étapes d'addition d'un nucléotide est effectuée en utilisant un dérivé de nucléoside tel que défini dans une quelconque des revendications 1 à 5, et **en ce que** la coupure du groupe protecteur de la fonction 5'-OH du nucléotide est effectuée par réaction électrochimique.

8. Procédé selon la revendication 7, **caractérisé en ce que** le support solide est constitué par au moins une électrode recouverte d'un polymère conducteur électronique.

9. Procédé selon une quelconque des revendications 7 ou 8, **caractérisé en ce que** le support solide est constitué par plusieurs électrodes juxtaposées, la déprotection électrochimique de la fonction 5'-OH pouvant, si on le souhaite, n'être effectuée que sur certaines de ces électrodes.

10. Procédé selon une quelconque des revendications 7 à 9 **caractérisé en ce que** la déprotection électrochimique de la fonction 5'-OH est effectuée en appliquant à l'électrode portant le support solide de synthèse une différence de potentiel, comprise entre -1,6 V et -0,7 V par rapport à une électrode de référence Ag/AgNO₃.

11. Procédé selon une quelconque des revendications 7 à 10, caractérisé en ce l'on effectue, après chaque étape de couplage d'un nucléotide, un traitement par l'anhydride isobutyrique.

## Patentansprüche

1. Verwendung, in einem Verfahren zur Synthese von Oligonukleotiden mit wenigstens einem elektrochemischen Entschützungsschritt und vor diesem Entschützungsschritt, wenigstens eines Nukleosid-Derivats entsprechend der Formel (I) : 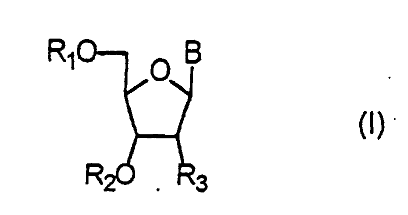 worin
- R₁ bedeutet:
* eine elektrolabile Gruppe entsprechend der folgenden Formel (IIa): 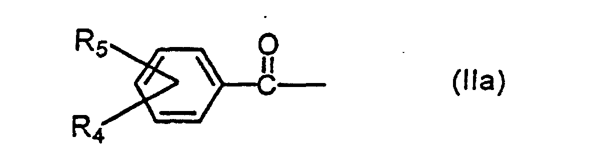 worin R₄ eine NO₂-Gruppe bedeutet und R₅ ein Wasserstoffatom, eine lineare oder verzweigte C₁-C₁₀-Alkylgruppe, eine Gruppe 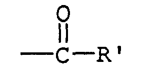 oder eine Gruppe 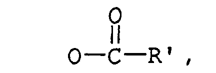 wobei R' eine lineare oder verzweigte C₁-C₁₀-Alkylgruppe ist, oder eine Gruppe ―O―R', wobei R' eine lineare oder verzweigte C₁-C₁₀-Alkylgruppe ist, bedeutet, oder eine elektrolabile Gruppe entsprechend der folgenden Formel (IIb): 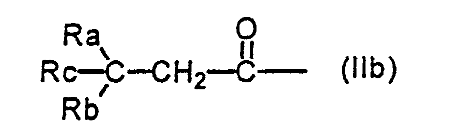 worin eine der Gruppen Ra, Rb oder Rc eine Phenylgruppe oder eine substituierte Phenylgruppe bedeutet und die beiden anderen Wasserstoffatome bedeuten;
- R₂ ein Wasserstoffatom oder eine Phosphoramidit-, Phosphonat- oder Phosphotriester-Gruppe bedeutet;
- R₃ ein Wasserstoffatom, einen Hydroxyrest, einen substituierten Hydroxyrest oder ein Halogen bedeutet;
- B einen von einer Purin- oder Pyrimidinbase abgeleiteten Rest gemäß einer der folgenden Formeln bedeutet: 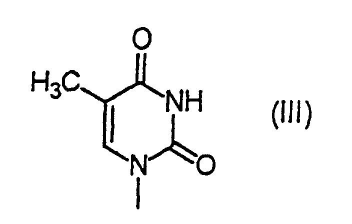 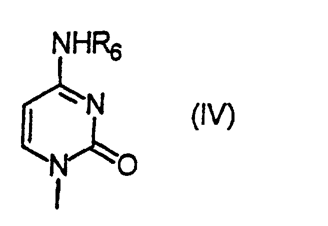 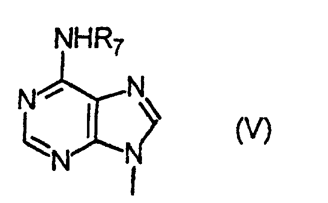 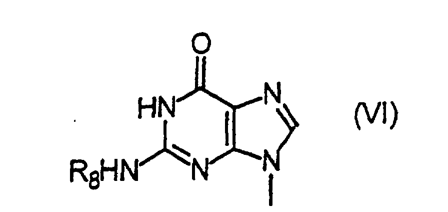 worin R₆ eine von einer Isobutyryl- oder Phenylpropionylgruppe unterschiedliche Schutzgruppe für die Aminfunktion entsprechend einer der folgenden Formeln (VII), (VIII) und (IX) bedeutet: 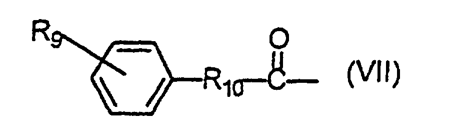 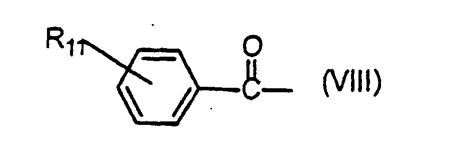 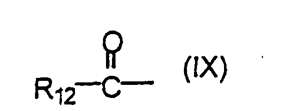 worin R₉ und R₁₁, die gleich oder verschieden sein können, ein Wasserstoffatom oder eine C₁-C₁₀-Alkyloder -Alkoxygruppe bedeuten, R₁₂ einen linearen oder verzweigten C₁-C₁₀-Alkylrest bedeutet und R₁₀ ausgewählt ist aus den Resten der Formel:
-CH₂-; 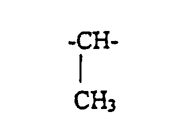 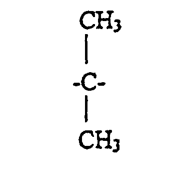
-O-(CH₂)- 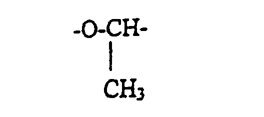 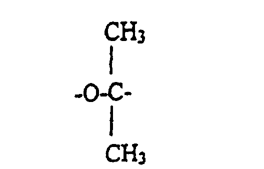
* R₇ eine von einer Phenylpropionylgruppe unterschiedliche Schutzgruppe für die Aminfunktion entsprechend einer der oben definierten Formeln (VII), (VIII) und (IX) bedeutet;
* R₈ eine von einer Isobutyryl-, Phenylpropionyloder Phenoxyacetylgruppe unterschiedliche Schutzgruppe für die Aminfunktion entsprechend einer der oben definierten Formeln (VII), (VIII) und (IX) bedeutet;
ausgenommen Derivate, bei denen R₁ eine elektrolabile Gruppe der Formel (IIa) ist, in der R₄ eine NO₂-Gruppe und R₅ ein Wasserstoffatom bedeutet und B der Formel (III) entspricht.

2. Verwendung nach Anspruch 1, **dadurch gekennzeichnet, daß** man ein Derivat einsetzt, bei dem R₁ der Formel (IIa) entspricht, in der R₄ eine NO₂-Gruppe bedeutet und R₅ ein Wasserstoffatom bedeutet.

3. Verwendung nach Anspruch 2, **dadurch gekennzeichnet, daß** man ein Derivat einsetzt, bei dem B der Formel (IV) entspricht, in der R₆ eine Anisoylgruppe ist.

4. Verwendung nach Anspruch 2, **dadurch gekennzeichnet, daß** man ein Derivat einsetzt, bei dem B der Formel (V) entspricht, in der R₇ eine Benzoylgruppe oder eine Phenoxyacetylgruppe bedeutet.

5. Verwendung nach Anspruch 2, **dadurch gekennzeichnet, daß** man ein Derivat einsetzt, bei dem B der Formel (VI) entspricht, in der R₈ eine tert-Butylphenoxyacetylgruppe bedeutet.

6. Nukleosid-Derivat wie in irgendeinem der Ansprüche 1 bis 5 definiert, ausgenommen Derivate, worin R₁ eine elektrolabile Gruppe der Formel (IIa) ist, in der R₄ eine NO₂-Gruppe und R₅ ein Wasserstoffatom bedeutet und B entweder der Formel (III) oder der Formel (IV) entspricht, in der R₆ eine Benzoylgruppe ist.

7. Verfahren zur Synthese von Oligonukleotiden durch sukzessive Zugabe von Nukleotiden ausgehend von einem ersten Nukleosid, das an einen festen Träger gebunden ist, wobei jede Zugabe eines Nukleotids die Abspaltung der Schutzgruppe von der 5'-OH-Funktion des Nukleotids oder des vorangehenden Nukleosids und die Kupplung des zuzugebenden Nukleotids an diese entschützte 5'-OH-Funktion umfaßt, wobei das Verfahren **dadurch gekennzeichnet ist, daß** wenigstens einer der Zugabeschritte für ein Nukleotid durchgeführt wird, indem ein Nukleosid-Derivat eingesetzt wird, wie es in irgendeinem der Ansprüche 1 bis 5 definiert ist, und daß die Abspaltung der Schutzgruppe von der 5'-OH-Funktion des Nukleotids durch elektrochemische Reaktion erfolgt.

8. Verfahren nach Anspruch 7, **dadurch gekennzeichnet, daß** der feste Träger aus wenigstens einer Elektrode besteht, die mit einem elektrisch leitfähigen Polymer überzogen ist.

9. Verfahren nach irgendeinem der Ansprüche 7 oder 8, **dadurch gekennzeichnet, daß** der feste Träger aus mehreren nebeneinander angeordneten Elektroden besteht, wobei die elektrochemische Entschützung der 5'-OH-Funktion, falls gewünscht, nur an bestimmten dieser Elektroden erfolgen kann.

10. Verfahren nach irgendeinem der Ansprüche 7 bis 9, **dadurch gekennzeichnet, daß** die elektrochemische Entschützung der 5'-OH-Funktion erfolgt, indem an die Elektrode, die den festen Träger für die Synthese trägt, eine Potentialdifferenz gegenüber einer Ag/AgNO₃-Referenzelektrode von zwischen -1,6 V und -0,7 V angelegt wird.

11. Verfahren nach irgendeinem der Ansprüche 7 bis 10, **dadurch gekennzeichnet, daß** man nach jedem Kupplungsschritt für ein Nukleotid eine Behandlung mit Isobuttersäureanhydrid durchführt.

## Claims

1. Use, in a process for synthesising oligonucleotides comprising at least one step of electrochemical deprotection, and prior to said step of deprotection, of at least one nucleoside derivative corresponding to formula I: 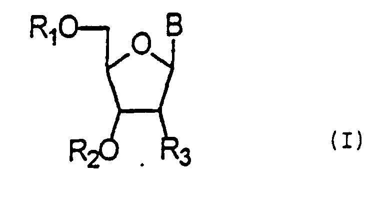 wherein
R1 denotes:
* an electrically unstable group corresponding to the following formula (IIa): 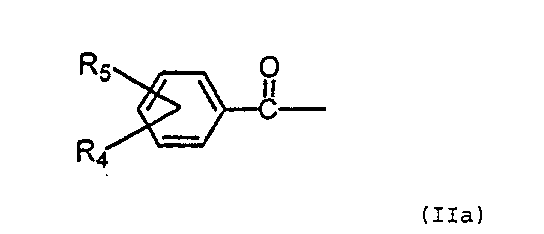 wherein R4 denotes an NO₂ group and R5 denotes a hydrogen atom, a straight-chain or branched C₁ to C₁₀ alkyl group, a group 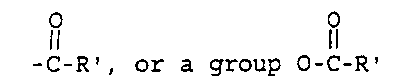 where R' denotes a straight-chain or branched C₁ to C₁₀ alkyl group, or a group -O-R' where R' denotes a straight-chain or branched C₁ to C₁₀ alkyl group, or an electrically unstable group corresponding to the following formula (IIb) : 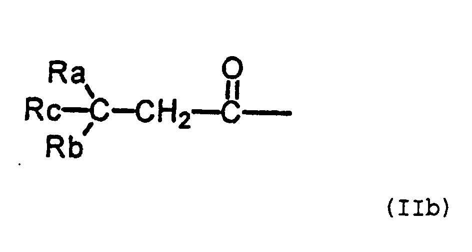 wherein one of the groups Ra, Rb or Rc denote a phenyl group or a substituted phenyl group, and the other two denote hydrogen atoms
R2 denotes a hydrogen atom or a phosphoroamidite, phosphonate or phosphotriester group;
R3 denotes a hydrogen atom, a hydroxyl radical, a substituted hydroxyl radical or a halogen;
B denotes a radical derived from a purine or pyrimidine base corresponding to one of the following formulae: 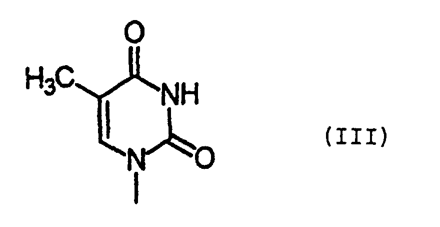 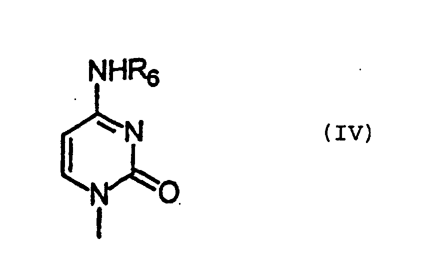 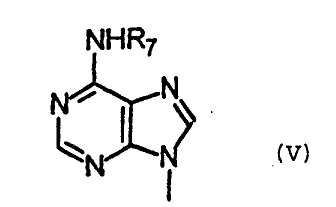 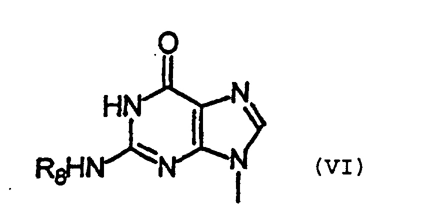 where R6 denotes a protecting group for the amine function, other than an isobutyryl or phenylpropionyl group, corresponding to one of the following formulae (VII), (VIII) and (IX): 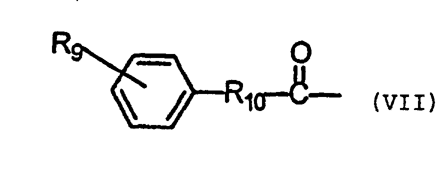 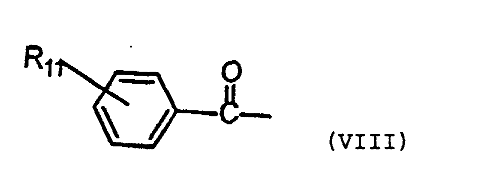 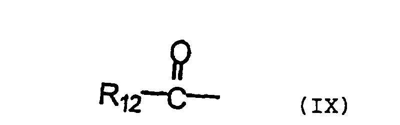 wherein R9 and R11, which may be identical or different, denote a hydrogen atom, or a C₁-C₁₀ alkyl or alkoxy group, R12 denotes a straight-chain or branched C₁-C₁₀ alkyl radical, and R10 is selected from the radicals of formulae:
-CH₂- ; 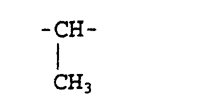 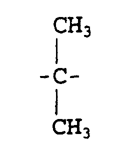
-O-(CH₂)- 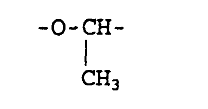 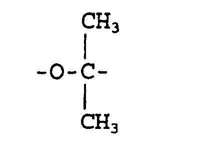
* R7 denotes a protecting group for the amine function, other than a phenylpropionyl group, corresponding to one of formulae (VII), (VIII) and (IX) defined above;
* R8 denotes a protecting group for the amine function, other than an isobutyryl, phenylpropionyl or phenoxyacetyl group, corresponding to one of the formulae (VII), (VIII) and (IX) defined above:
with the exception of the derivatives wherein R1 is an electrically unstable group of formula (IIa) in which R4 denotes an NO₂ and R5 is a hydrogen atom, and B corresponds to formula III.

2. Use according to claim 1, **characterised in that** a derivative is used wherein R1 corresponds to formula (IIa), wherein R4 denotes an NO₂ group and R5 denotes a hydrogen atom.

3. Use according to claim 2, **characterised in that** a derivative is used wherein B corresponds to formula (IV) in which R6 is an anisoyl group.

4. Use according to claim 2, **characterised in that** a derivative is used wherein B corresponds to formula (V) wherein R7 is a benzoyl group or a phenoxyacetyl group.

5. Use according to claim 2, **characterised in that** a derivative is used wherein B corresponds to formula (VI) wherein R8 is a *t*-butylphenoxyacetyl group.

6. Nucleoside derivative as defined in any one of claims 1 to 5, with the exception of the derivatives in which R1 is an electrically unstable group of formula (IIa) wherein R4 denotes an NO₂ group and R5 denotes a hydrogen atom, and B corresponds either to formula (III) or to formula (IV) wherein R6 is a benzoyl group.

7. Process for synthesising oligonucleotides by the successive addition of nucleotides starting from a first nucleoside fixed to a solid support, each addition of a nucleotide comprising cleaving the protecting group from the 5'-OH function of the previous nucleotide or nucleoside, and the coupling of the nucleotide to be added to said deprotected 5'-OH function, the process being **characterised in that** at least one of the steps of adding a nucleotide is carried out using a nucleoside derivative as defined in any one of claims 1 to 5, and **in that** the cleaving of the protecting group from the 5'-OH function of the nucleotide is carried out by electrochemical reaction.

8. Process according to claim 7, **characterised in that** the solid support consists of at least one electrode covered with a polymer which is an electronic conductor.

9. Process according to one of claims 7 or 8, **characterised in that** the solid support is made up of a number of juxtaposed electrodes, the electrochemical deprotection of the 5'-OH function possibly being carried out, if desired, on only some of these electrodes.

10. Process according to any one of claims 7 to 9, **characterised in that** the electrochemical deprotection of the 5'-OH function is carried out by applying, to the electrode which carries the solid support for the synthesis, a difference in potential of between -1.6 V and -0.7 V, compared with an Ag/AgNO₃ reference electrode.

11. Process according to any one of claims 7 to 10, **characterised in that** after each step of coupling a nucleotide a treatment is carried out with isobutyric anhydride.
